# EUROPEAN PATENT APPLICATION

(11) **EP 4 550 342 A1**
(43) Date of publication of application: **07.05.2025**
(21) Application number: 24209658.4
(22) Date of filing: 29.10.2024
(51) Int. Cl.: G16H 10/60, A61B 5/00, G16H 40/67

(54) **CENTRAL MONITORING STATION WITH SHARED VIDEO MEMORY**

(30) Priority: 01.11.2023 US 202363546826 P
(71) Applicant: Draeger Medical Systems, Inc., Andover, MA 01810 (US)
(72) Inventor: RISHER-KELLY, Clifford M., Wells, 04090 (US)
(74) Representative: Haseltine Lake Kempner LLP

(57) **Abstract**

A central monitoring station, particularly for use in a clinical setting includes a medical data information base ("MDIB") buffer for receiving and storing real-time MDIB patient data from a plurality of patient monitors. A software module for creates visualizations of the MDIB patient data stored in the MDIB buffer and stores the visualizations of the MDIB patient data in a central video memory. A remote video distribution device retrieves selected visualizations of MDIB patient data from the central video memory and generates a video output signal. The video output signal may be provided to a video display of the central monitoring station and/or to one or more of the plurality of patient monitors.

## Description

### BACKGROUND

The present disclosure relates generally to patient monitoring systems, and particularly to monitoring systems utilized in clinical settings and including video display of patient monitoring waveforms and other patient data.

This section of this document introduces information about and/or from the art that may provide context for or be related to the subject matter described herein and/or claimed below. It provides background information to facilitate a better understanding of the various aspects of the present invention. This is a discussion of "related" art. That such art is related in no way implies that it is also "prior" art. The related art may or may not be prior art. The discussion in this section of this document is to be read in this light, and not as admissions of prior art.

Patient monitors are devices that are configured to receive physiological data from another device and either display a patient's physiological data, monitor a patient's physiological data, or both. A patient monitor may be configured to be worn by a patient, may be a hand-held device, may be docked to or undocked from a larger unit such as a monitor mount, and, thus, may be transportable. For example, a monitor mount may be a larger patient monitor or a console that has a docking interface or docking receptacle to which the patient monitor can be removably docked.

Some patient monitors support both a local view and a remote view on a display. A local view pertains to displaying a physiological data of a patient that is located locally with respect to the patient monitor. In other words, in a local view, a patient monitor displays physiological data obtained by sensors, medical devices, and/or therapy devices that interface with a local patient. The patient monitor itself is located proximate to the local patient (e.g., in the same room or bedside) and can be said to be assigned to the local patient. In contrast, a remote view pertains to displaying a physiological data of a patient that is located remotely with respect to the patient monitor. In other words, physiological data of a remote patient is obtained from a different, remote patient monitor and the patient monitor may be located remote from a remote patient (e.g., in a different room or bedside). Thus, a remote view enables a clinician to view a visual representation of another patient monitor on a local patient monitor.

### SUMMARY

A patient monitor that supports both local and remote views can display physiological data of a local patient and of one or more remote patients by, for example, a split screen, screen overlays, and the like, with each portion of a screen dedicated to a different patient. So-called "alarm groups" may be established for individual nurses/clinicians who are assigned responsibility for a subset of the overall number of patients in a given facility. For example, without limitation, a clinical setting such as a hospital may serve 100 patients and have twenty individual nurses or clinicians. In such a scenario, each nurse/clinician would be responsible for five patients. Thus, an alarm group including those five patients would be defined for each of the nurses/clinicians.

A patient monitor may be configured into an Actionable Bed-to-Bed Alarm mode for providing a remote view of another patient monitor. Because a patient monitor is often assigned to a bed, and thus to a specific patient residing in that bed, a patient monitor may be referred to as a "bed." A bed may be used as a generic term for a ventilator machine, an anesthesia machine, or any other device that is assigned to a specific local location or patient. A patient monitor configured into Actionable Bed-to-Bed Alarm mode is configured to provide an alarm generated by a remote patient monitor to a clinician in real-time so that the clinician can determine if an emergency situation is occurring at a remote bed (i.e., at a remote patient monitor and specifically at a remote patient). In this case, the latency of the connection between patient monitors and displaying or rendering the physiological data of the remote patient on the screen of the local patient monitor is important to the function.

In past generations of patient monitors, multi-cast was used on the network as a means for each monitor to broadcast their waveforms and parameters to all consumers on the network. This multi-cast communication included connections to a central station, a gateway, and other patient monitors that were configured for remote viewing of this data. With the new communication standard of service-oriented device connectivity ("SDC"), multicast is not permitted due to security reasons, and all data must be sent with point-to-point connections. Multicast is group communication where data transmission is addressed to a group of destination computers simultaneously. In contrast, SDC is a web services-based architecture that enables interoperability amongst point-of-care medical devices and data exchange between point-of-care devices and Health Level Seven ("HL7") compatible clinical and hospital information systems. In other words, SDC provides separate point-to-point connections to each device. It enables a hospital's medical technologies to share data and information bi-directionally, securely, and dynamically.

The process of making an SDC connection and generating waveforms and alarms on the screen using the platform software is too slow for the Actionable Bed-to-Bed Alarm functionality. In addition, supporting the possible number of SDC connections required for a care group is not feasible with the present CPU power of a patient monitor. Therefore, a device capable of providing a remote view cluster service that supports SDC connections between devices with low latency may be desired.

There exists a need for improved methods and systems for providing patient monitoring and alarm systems for a plurality of patients in a clinical setting in which latency between acquisition of patient monitor data and the display of the patient monitoring data among a given patient within a given alarm group or across a plurality of alarm groups is minimized.

To resolve or mitigate at least one or more of the above problems and potentially other present or future problems, one aspect of the present disclosure relates to providing a shared, central video memory for storing visualizations of patient data acquired by a plurality of individual patient monitors. The patient monitor data is first stored in a data buffer, and is thereafter processed to create visualizations of the patient monitor data that are stored in a shared central video memory. Selected visualizations stored in the shared central video memory may then be provided both to a display of the central monitoring system and/or to one or more individual patient monitors, which may be grouped in alarm groups which include a subset of the plurality of patient monitors.

Further, in examples, because the patient data is stored in real-time in a shared, central video memory, when a remote view of patient data is activated at a patient monitor remote from the central station, the patient data from a previous time period, e.g., ten seconds prior, may be immediately displayed at the patient monitor, because it is immediately available from the shared, central video memory. This avoids a delay involved in incrementally building a waveform display from stored data points once a remote view is activated.

One or more examples in the present disclosure provide but are not limited to the advantages noted hereinbelow. Note that not all embodiments will necessarily manifest all of these advantages. Furthermore, to the extent that one or more embodiments manifest one or more of the advantages, not all embodiments will manifest such advantages to the same extent or degree.

The above presents a simplified summary in order to provide a basic understanding of some aspects of what is claimed below. This summary is not an exhaustive overview of the claimed subject matter. It is not intended to identify key or critical elements of the disclosure or to delineate the scope of the claims. Its sole purpose is to present some concepts in a simplified form as a prelude to the more detailed description that is discussed later.

### BRIEF DESCRIPTION OF THE DRAWINGS

In the drawings, like reference numbers generally indicate identical, functionally similar, and/or structurally similar elements.
FIG. 1 illustrates a patient monitoring system according to one or more examples;
FIG. 2 is a block diagram illustrating a central patient monitoring station incorporating a central video memory according to one or more examples;
FIG. 3 is a block diagram illustrating a central patient monitoring station incorporating a central video memory according to one or more alternative examples;
FIG. 4 is a block diagram illustrating a central patient monitoring station incorporating a central video memory according to one or more alternative examples;
FIG. 5 is a block diagram illustrating a central patient monitoring station incorporating a central video memory according to one or more alternative examples;
FIG. 6 is a block diagram illustrating a central patient monitoring station incorporating a central video memory according to one or more alternative examples;
FIG. 7 is a flow diagram illustrating a method of acquiring and displaying patient data according to one or more examples; and
FIG. 8 is a block diagram illustrating a computing resource implementing a method of operating a central monitoring system according to one or more examples.

While the disclosed subject matter is susceptible to various modifications and alternative forms, the drawings illustrate specific implementations described in detail by way of example. It should be understood, however, that the description herein of specific examples is not intended to limit that which is claimed to the particular forms disclosed, but on the contrary, the intention is to cover all modifications, equivalents, and alternatives falling within the spirit and scope of the appended claims.

### DETAILED DESCRIPTION

Illustrative examples of the subject matter claimed below are disclosed. In the interest of clarity, not all features of an actual implementation are described for every example in this specification. It will be appreciated that in the development of any such actual implementation, numerous implementation-specific decisions may be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

The expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component or a combination thereof, but should not be construed to exclude the existence of or a possibility of the addition of one or more other characteristics, numbers, operations, constituent elements, components or combinations thereof.

As used herein, the article "a" is intended to have its ordinary meaning in the patent arts, namely "one or more." Herein, the term "about" when applied to a value generally means within the tolerance range of the equipment used to produce the value, or in some examples, means plus or minus 10%, or plus or minus 5%, or plus or minus 1%, unless otherwise expressly specified. Further, herein the term "substantially" as used herein means a majority, or almost all, or all, or an amount with a range of about 51 % to about 100%, for example. Moreover, examples herein are intended to be illustrative only and are presented for discussion purposes and not by way of limitation.

As used herein, to "provide" an item means to have possession of and/or control over the item. This may include, for example, forming (or assembling) some or all of the item from its constituent materials and/or, obtaining possession of and/or control over an already-formed item.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted. In the following, details are set forth to provide a more thorough explanation of the embodiments. However, it will be apparent to those skilled in the art that embodiments may be practiced without these specific details. In other instances, well-known structures and devices are shown in block diagram form or in a schematic view rather than in detail in order to avoid obscuring the embodiments. In addition, features of the different embodiments described hereinafter may be combined with each other, unless specifically noted otherwise. For example, variations or modifications described with respect to one of the embodiments may also be applicable to other embodiments unless noted to the contrary.

Further, equivalent or like elements or elements with equivalent or like functionality are denoted in the following description with equivalent or like reference numerals. As the same or functionally equivalent elements are given the same reference numbers in the figures, a repeated description for elements provided with the same reference numbers may be omitted. Hence, descriptions provided for elements having the same or like reference numbers are mutually exchangeable.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," *etc*.).

In the present disclosure, expressions including ordinal numbers, such as "first", "second", and/or the like, may modify various elements. However, such elements are not limited by the above expressions. For example, the above expressions do not limit the sequence and/or importance of the elements. The above expressions are used merely for the purpose of distinguishing an element from the other elements. For example, a first box and a second box indicate different boxes, although both are boxes. For further example, a first element could be termed a second element, and similarly, a second element could also be termed a first element without departing from the scope of the present disclosure.

A sensor refers to a component which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

ECG signal processing, as used herein, refers to, without limitation, manipulating an analog signal in such a way that the signal meets the requirements of a next stage for further processing. ECG signal processing may include converting between analog and digital realms (e.g., via an analog-to-digital or digital-to-analog converter), amplification, filtering, converting, biasing, range matching, isolation and any other processes required to make a sensor output suitable for processing.

The term "real-time," as used herein, refers to systems and processes which controls or operates on an environment by receiving data, processing data, and/or returning the results sufficiently quickly to affect the environment at that time, taking into account the inherent latencies of electrical signal propagation. For example, the "real-time" display of an ECG signal or other physiological measurement on a patient monitor may have an intrinsic delay on the order of milliseconds between the actual ECG activity and a displayed waveform, as the ECG signal is conducted on one or more ECG leads to circuitry for filtering and/or amplification prior to being displayed. In cases involving the display of patient physiological data on a patient monitor, as described herein, a delay of less than one-half second may be considered "real-time."

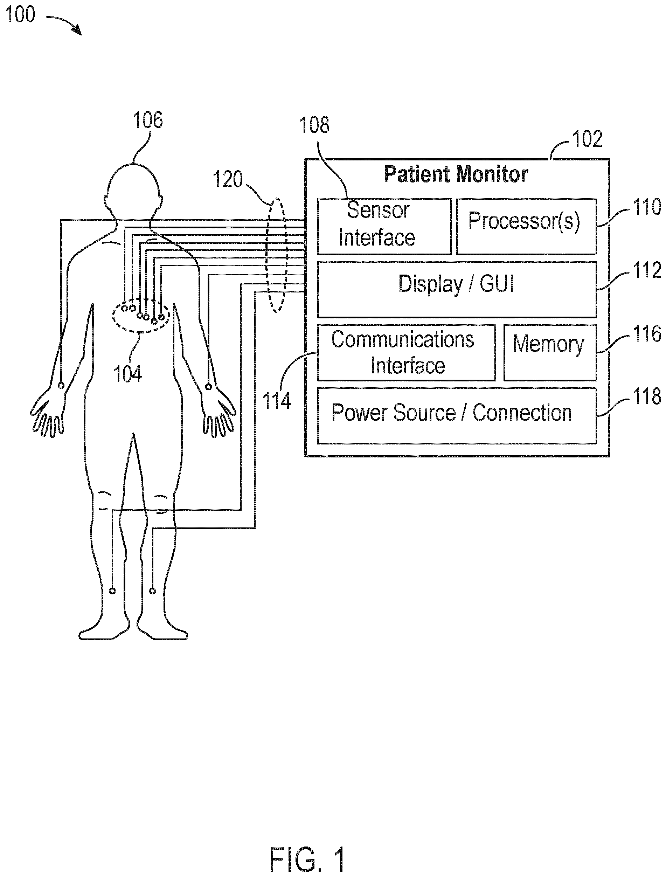

Turning now to the drawings, FIG. 1 shows a physiological monitoring system 100 according to one or more examples. As shown in FIG. 1, the physiological monitoring system 100 includes a patient monitor 102 (e.g., a physiological monitoring device) capable of receiving physiological data from various sensors 104 connected to a patient 106. In this example, sensors 104 comprise ECG electrodes affixed to the skin of patient 106.

In general, it is contemplated by the present disclosure that patient monitor 102 includes electronic components and/or electronic computing devices operable to receive, transmit, process, store, and/or manage patient data and information associated performing the functions of the system as described herein, which encompasses any suitable processing device adapted to perform computing tasks consistent with the execution of computer-readable instructions stored in a memory or a computer-readable recording medium.

Further, any, all, or some of the computing devices in patient monitor 102 may be adapted to execute any operating system, including Linux^{®}, UNIX^{®}, Windows Server^{®}, *etc.,* as well as virtual machines adapted to virtualize execution of a particular operating system, including customized and proprietary operating systems. Patient monitor 102 may be further equipped with components to facilitate communication with other computing devices over one or more network connections, which may include connections to local and wide area networks, wireless and wired networks, public and private networks, and any other communication network enabling communication in the system.

As shown in FIG. 1, patient monitor 102 may be, for example, a patient monitor implemented to monitor various physiological parameters of patient 106 via sensors 104. Patient monitor 102 may include a sensor interface 108, one or more processors 110, a display/graphical user interface ("GUI") 112, a communications interface 114, a memory 116, and a power source (or power connection) 118. Sensor interface 108 may be implemented in hardware or combination of hardware and software and is used to connect via wired and/or wireless connections to sensors 104 for gathering physiological data from the patient 106. As noted, sensors 104 in the present example are ECG electrodes affixed to the skin of patient 106. A plurality of conductive leads 120, comprising a plurality of conductive cables, are provided for coupling sensors 104 to sensor interface 108. In one or more examples, conductive leads 120 comprise a plurality of ECG cables.

The data signals from the sensors 104 may include, for example, sensor data related to an ECG. The one or more processors 110 may be used for controlling the general operations of patient monitor 102, as well as processing sensor data received by sensor interface 108. The one or more processors 110 may be, but are not limited to, a central processing unit ("CPU"), a hardware microprocessor, a multi-core processor, a single core processor, a field programmable gate array ("FPGA"), a microcontroller, an application specific integrated circuit ("ASIC"), a digital signal processor ("DSP"), or other similar processing device capable of executing any type of instructions, algorithms, or software for controlling the operation and performing the functions of patient monitor 102. In some embodiments, the one or more processors 110 may comprise a processor chipset including, for example and without limitation, one or more co-processors.

Display/GUI 112 may be configured to display various patient data, sensor data, and hospital or patient care information, and includes a user interface implemented for allowing interaction and communication between a user and patient monitor 102. Display/GUI 112 may include a keyboard (not shown) and/or pointing or tracking device (not shown), as well as a display, such as a liquid crystal display ("LCD"), cathode ray tube ("CRT") display, thin film transistor ("TFT") display, light-emitting diode ("LED") display, high definition ("HD") display, or other similar display device that may include touch screen capabilities. Display/GUI 112 may provide a means for inputting instructions or information directly to the patient monitor 102. The patient information displayed may, for example, relate to the measured physiological parameters of patient 106 (*e.g.,* ECG readings).

Communications interface 114 may enable patient monitor 102 to directly or indirectly (via, for example, a monitor mount) communicate with one or more computing networks and devices, workstations, consoles, computers, monitoring equipment, alert systems, and/or mobile devices (*e.g.,* a mobile phone, tablet, or other hand-held display device). Communications interface 114 may include various network cards, interfaces, communication channels, cloud, antennas, and/or circuitry to enable wired and wireless communications with such computing networks and devices. Communications interface 114 may be used to implement, for example, a Bluetooth^{®} connection, a cellular network connection, and/or a WiFi^{®} connection with such computing networks and devices. Example wireless communication connections implemented using the communication interface 114 include wireless connections that operate in accordance with, but are not limited to, IEEE802.11 protocol, a Radio Frequency For Consumer Electronics ("RF4CE") protocol, and/or IEEE802.15.4 protocol (*e.g.,* ZigBee^{®} protocol). In essence, any wireless communication protocol may be used.

Additionally, communications interface 114 may enable direct (*i.e.,* device-to-device) communications (*e.g.,* messaging, signal exchange, *etc*.) such as from a monitor mount to patient monitor 102 using, for example, a universal serial bus ("USB") connection or other communication protocol interface. The communication interface 114 may also enable direct device-to-device connection to other devices such as to a tablet, computer, or similar electronic device; or to an external storage device or memory.

Memory 116 may be a single memory device or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. Memory 116 may be used to store any type of instructions and patient data associated with algorithms, processes, or operations for controlling the general functions and operations of patient monitor 102.

Power source 118 may include a self-contained power source such as a battery pack and/or include an interface to be powered through an electrical outlet (either directly or by way of a monitor mount). Power source 118 may also be a rechargeable battery that can be detached allowing for replacement. In the case of a rechargeable battery, a small built-in back-up battery (or super capacitor) can be provided for continuous power to be provided to patient monitor 102 during battery replacement. Communication between the components of patient monitor 102 in this example (may be established using an internal bus (not explicitly shown in FIG. 1).

Patient monitor 102 may be attached to one or more of several different types of sensors 104 and may be configured to measure and readout physiological data related to patient 106. As noted, sensors 104 may be attached to patient monitor 102 by conductive leads 120 which may be, for example, cables coupled to sensor interface 108. Additionally, or alternatively, one or more sensors 104 may be connected to sensor interface 108 via a wireless connection. In which case sensor interface 108 may include circuity for receiving data from and sending data to one or more devices using, for example, a WiFi^{®} connection, a cellular network connection, and/or a Bluetooth^{®} connection.

The data signals received from sensors 104, may be analog signals. For example, the data signals for the ECG may be input to sensor interface 108, which can include an ECG data acquisition circuit (not shown separately in FIG. 1). An ECG data acquisition circuit may include amplifying and filtering circuity as well as analog-to-digital (A/D) circuity that converts the analog signal to a digital signal using amplification, filtering, and A/D conversion methods. In the event that the ECG sensor is a wireless sensor, sensor interface 108 may receive the data signals from a wireless communication module. Thus, sensor interface 108 is a component which may be configured to interface with one or more sensors 104 and receive sensor data therefrom.

As further described herein, the processing performed by an ECG data acquisition circuit may generate analog data waveforms or digital data waveforms that are analyzed by, in this particular embodiment, a microcontroller. However, other embodiments may use other kinds of processors. The microcontroller may be one of the processors 110.

The one or more processors 110, for example, may analyze the ECG waveforms to identify certain waveform characteristics and threshold levels indicative of conditions (abnormal and normal) of the patient 106 using one or more monitoring methods. A monitoring method may include comparing an analog or a digital waveform characteristic or an analog or digital value to one or more threshold values and generating a comparison result based thereon. The microcontroller may be, for example, a processor, an FPGA, an ASIC, a DSP, a microcontroller, or similar processing device. The microcontroller may include a memory or use a separate memory 116. The memory may be, for example, a RAM, a memory buffer, a hard drive, a database, an EPROM, an EEPROM, a ROM, a flash memory, a hard disk, or any other non-transitory computer readable medium.

Memory 116 may store software or algorithms with executable instructions and the microcontroller may execute a set of instructions of the software or algorithms in association with executing different operations and functions of patient monitor 102 such as analyzing the digital data waveforms related to the data signals from sensors 104.

As noted, in the example of FIG. 1, conductive leads 120 between sensors 104 and sensor interface 108 may be an ECG lead set. Conductive leads 120 typically terminate at a sensor 104 that is attached to the patient for measuring ECG data.

As noted, an ECG signal reflects electrical impulses in the heart associated with the systolic rhythm of a beating heart. A normal sinus rhythm includes a repeating succession of "heartbeats" corresponding to heart contractions, and an ECG signal may reflect various phases of such contractions.

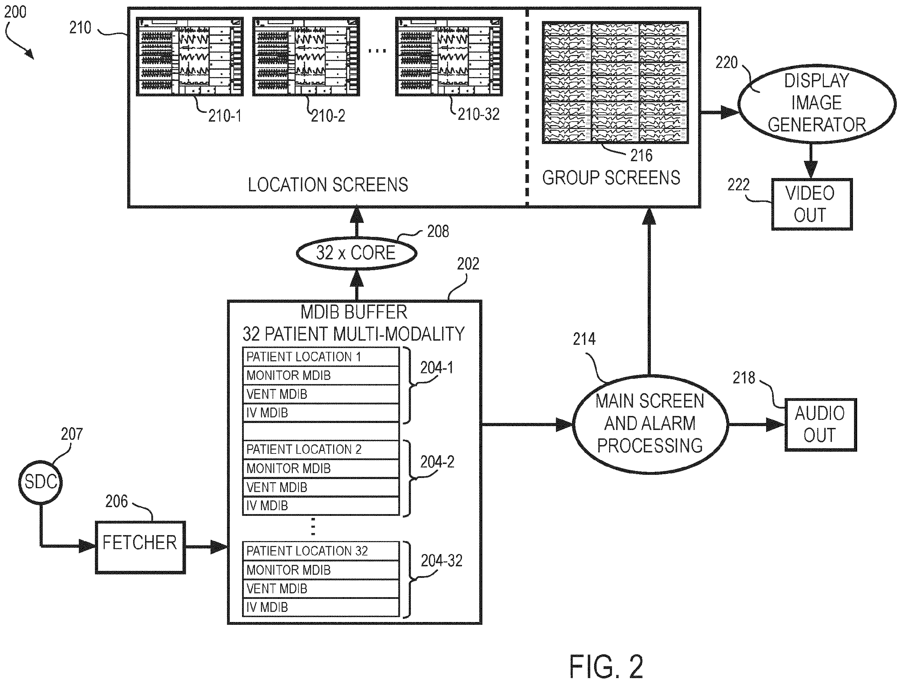

Turning to FIG. 2, there is shown a block diagram illustrating the architecture of a central monitoring station 200 according to one or more embodiments. In some embodiments, central monitoring station is a discrete computer platform including a processor, memory ("RAM"), removable and/or installed storage, and one or more displays and other user interface devices. In other embodiments, the functionality of central monitoring station 200 may be implemented as a virtual or "cloud" device accessed remotely over some kind of private or public computing system, such as a network.

As shown in FIG. 2, central monitoring station 200 includes an MDIB buffer memory 202 for storing a plurality of medical data information base ("MDIB") 204-1, 204-2, ... 204-32 (collectively, MDIBs 204). In the example of FIG. 2, MDIB buffer memory 202 has a capacity for thirty-two separate MDIBs, although buffer memories with greater or lesser capacity might be utilized depending upon the intended environment.

MDIBs 204 in MDIB buffer memory 202 are "multi-mode" MDIBs, insofar as each individual MDIB 204 includes a plurality of patient data streams. In examples, the patient data streams are fed to MDIB buffer memory 202 via a service-oriented device connectivity ("SDC") connection 207. SDC is a connection interface configured to be connected to patient monitoring devices, such as patient monitor 102 in FIG. 1. In the example of FIG. 2, each MDIB 204 stores an ECG monitor MDIB providing real-time ECG data, a ventilator MDIB providing real-time data relative to operation of a patient ventilator, and an IV MDIB providing real-time data relative to the condition of one or more intravenous ("IV") lines to a patient.

In the example of FIG. 2, MDIB data (waveforms and related information such as alarms) are supplied to MDIB buffer memory 202 from a fetcher 206, which may be connected either directly, *e.g.,* via a local area network, or wirelessly, to one or more patient monitors such as patient monitor 102 in the example of FIG. 1. Fetcher 206 is connected to SDC connection 207, receives MDIB data streams therefrom, and provides MDIB buffer memory 202 with real-time MDIB data streams from the individual patient monitors ("beds") serviced by central monitoring station 200.

In examples, data accumulated and aggregated in MDIB buffer memory 202 is fed to "core" software 208 which visualizes the MDIB data, including real-time waveforms and alarm information. In various embodiments, core software 208 is instantiated multiple times (*e.g.,* 32 times in the example of FIG. 2), in order to store and continuously update, in a central video memory 210, a real-time visualization of the bedside monitor data and alarm status from each bed serviced by central monitoring station 200. In FIG. 2, the visualized MDIB data for a plurality of location (bed) screens 212-1, 212-2, ... 212-32 are shown being stored in central video memory 210.

As further shown in FIG. 2, MDIB buffer memory 202 may be coupled to a main screen processing and alarm processing module 214 for providing group screen information to central video memory 210. A group screen portion 216 of central video memory 210 may be provided for providing a "cluster" of patient information views. Main screen and alarm processing module may also provide alarm notifications to an audio output module 218.

As further illustrated in FIG. 2, a display image generator module 220 provides a video output 222 for selectively displaying the content of central video memory 210 at a central station. User interface controls (not shown in FIG. 2 but to be described hereinbelow) may be utilized to selectively control which location screens and group screen are including in the central monitoring station video output 222.

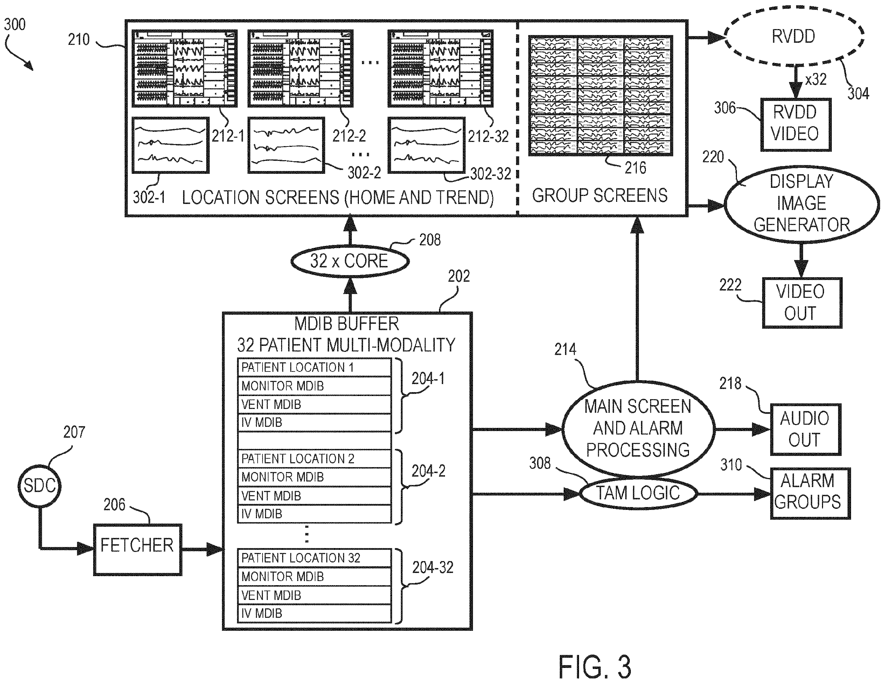

FIG. 3 is a block diagram of an alternative embodiment of a central monitoring station 300 according to one or more examples, in which elements which are functionally the same as those of the embodiment of FIG. 2 have retained identical reference numerals. In the embodiment of FIG. 3, in addition to visualizations of the bedside monitor data 212-1 ... 212-32 described above with reference to FIG. 2, central video memory 210 also stores a corresponding plurality of trend window visualizations 302-1 ... 302-32 which may provide longer-term trend visualizations (plots) of patient monitor data.

In addition, in the embodiment of FIG. 3, a remote video distribution device ("RVDD") 304 provides an RVDD video output signal 306 to each of the beds serviced by the central monitoring station 300. In examples, the video output signal to each bed serviced by the central monitoring station may be provided in real-time, *i.e.,* in less than one-half of a second. This functionality is achieved in part due to the use of central video memory 210, which maintains real-time updated visualized MDIB data. RVDD 304 selectively retrieves, based on predefined alarm group definitions, the visualized MDIB data stored in central video memory 210 and generates remote view MDIB visualizations therefrom to create custom remote views for each of the remote locations (beds) services by central monitoring station 300. To create a remote view MDIB for a particular bed, RVDD 304 needs only to create pointers to locations within central video memory 210 storing the visualized MDIB data corresponding to the patient monitor associated with the bed. RVDD then rapidly (*i.e.,* in one-half of a second or even less) provides the RVDD video output 306 to the associated patient monitor.

In examples, RVDD 304 may select MDIBs that correspond to remote patients (beds) that are assigned to a common alarm group, *e.g.,* the patients for which a particular nurse or clinician is responsible. For example, a patient monitor, such as patient monitor 102 shown in FIG. 1, may be connected to a first patient for a local view (*e.g.,* views 212-1 ... 212-32 stored in central video memory 210), and assigned to some number (*e.g.,* 3) other remote patients in an alarm group for a remote view on the first patient's local view. Once the pertinent MDIB visualizations stored in central video memory 210 are selected, RVDD 304 may select certain data objects from each of the MDIB visualizations for remote view depending upon remote view settings that may be preselected by the user and the state of alarms among the bed associated with the alarm group.

As further illustrated in FIG. 3, a targeted alarm manager ("TAM") module 308 may be provided to extract and decode alarm information from the MDIBs 204-1...204-32 stored in MDIB buffer memory 202 and provide an alarm group output 310, which may in some examples be in the form of textual data or alerts that are sent to one or more alarm dashboards (not shown) and/or that may be sent to individual remote monitors associated with beds in a particular alarm group.

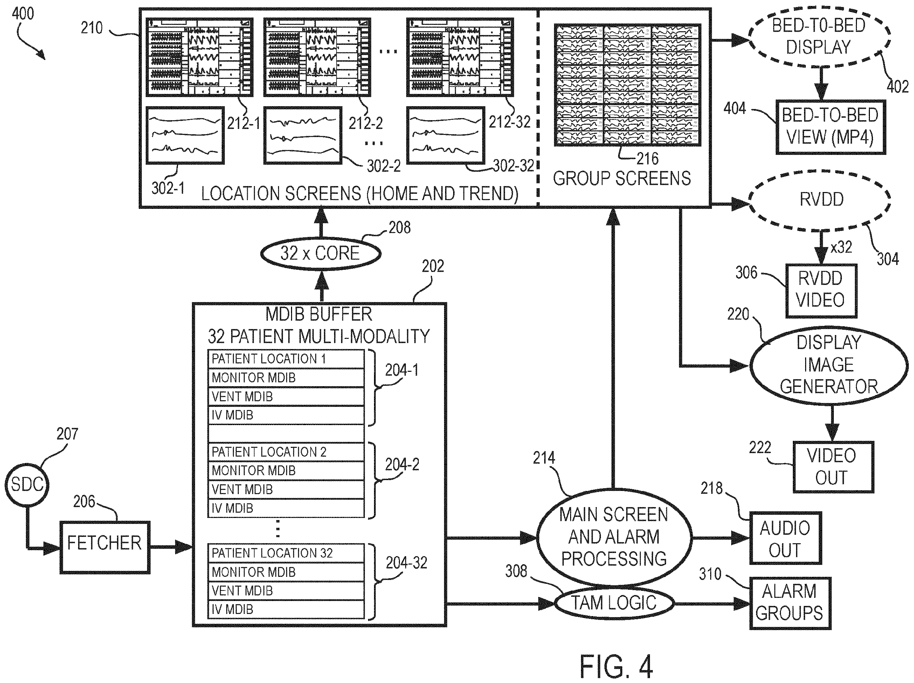

FIG. 4 is a block diagram of an alternative embodiment of a central monitoring station 400 according to one or more examples, in which elements which are functionally the same as those of the embodiments of FIG. 2 and 3 have retained identical reference numerals. In the example of FIG. 4, in addition to the display image provided at video output 222 at central station 200, and the RVDD video provided at output 306 to a patient monitor, central monitoring station 400 includes a bed-to-bed display module 402 for providing a bed-to-bed visualization 404 whereby alarms and displays generated by a remote patient monitor to a clinician if an emergency situation is occurring at remote beds, *i.e.,* at a remote patient monitor within a predefined alarm group.

In examples, the bed-to-bed functionality may provide the ability for a patient monitor in an alarm group to remotely display the MDIB visualizations not only of the patient with whom the monitor is associated, but also of one or more other MDIB visualizations from monitors in the alarm group. Thus, for example, when a nurse or clinician present at the bed of a first patient and second patient in the same alarm group as the first patient goes into an alarm status, the MDIB visualization of the second patient may be displayed alongside (or instead of) the MDiB visualization of the first patient.

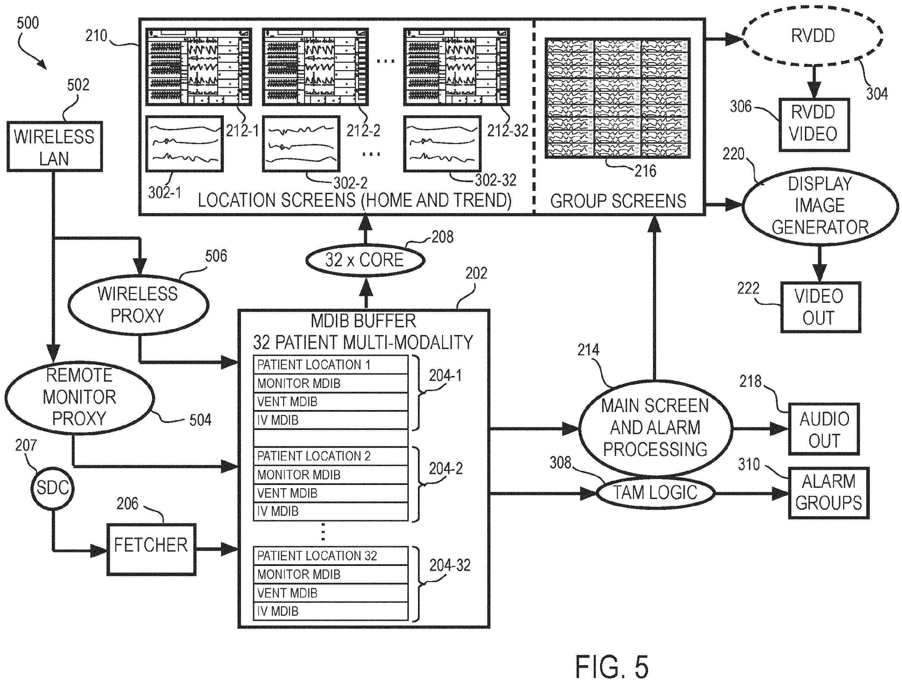

FIG. 5 is a block diagram of an alternative embodiment of a central monitoring station 500 according to one or more examples, in which elements which are functionally the same as those of the embodiments of FIG. 2, 3, and 4 have retained identical reference numerals. In the example of FIG. 5, a wireless local area network ("LAN") 502 is provided in the clinical setting for communicating with individual patient monitors.

In examples, patients may periodically be ambulatory, *i.e.,* separated from the bedside patient monitor. In such instances, the patient may be provided with a wireless remote patient monitor (not shown in the figures). The MDIB data from wireless remote patient monitors may be transmitted to wireless LAN 502. In the example of FIG. 5, central monitoring station 500 is connected to wireless LAN 502, and includes a remote monitor proxy module 504, which may be a software-implemented proxy module, for receiving transmitted patient data from patients' remote patient monitors. Due to bandwidth constraints, patient data from remote patient monitors may be formatted (*e.g.,* compressed or "packed") into a particular data format. Consequently, remote monitor proxy module 504 may translate the transmitted patient data into the SDC format and then provide the remote patient monitor data to MDIB buffer memory 202. Thereafter, the MDIB buffer memory 202 populates visualizations of the remote patient monitor data, through core software 208 within central video memory 210.

Similarly, as shown in FIG. 5, some bedside monitors such as patient monitor 102 shown in FIG. 1, may themselves have wireless telemetry capabilities, such that even if not connected directly to SDC connection 207, they may still transmit patient data to central monitoring station 500. For example, a bed-bound patient may be transported from one location to another within the clinical setting with the telemetry-capable bedside monitor still attached to the patient. In such cases, a wireless proxy module 506, which may also be a software-implemented proxy module, may receive telemetry from wireless LAN 502 and translate the wireless patient data and provide it to MDIB buffer memory 202. Thereafter, visualizations of the patient data are provided, through core software 208, to central video memory 210.

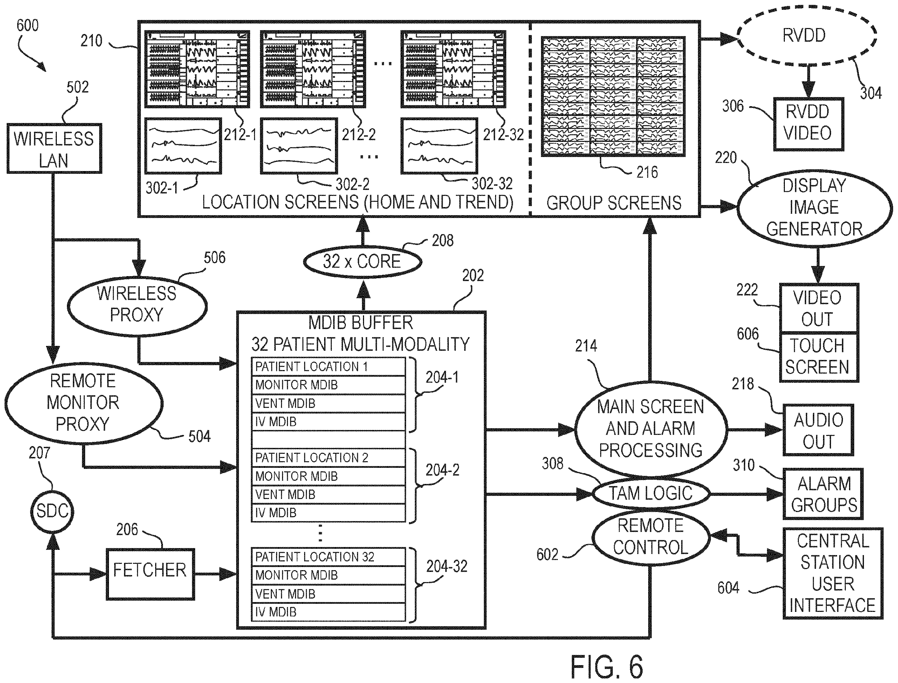

FIG. 6 is a block diagram of an alternative embodiment of a central monitoring station 600 according to one or more examples, in which elements which are functionally the same as those of the embodiments of FIG. 2, 3, 4, and 5 have retained identical reference numerals. In the example of FIG. 6, a remote control module is provided in central monitoring station 600 for enabling users located at the central monitoring station 600 to exercise control over the plurality of patient monitors coupled via SDC connection 207 to central monitoring station. Using a central station user interface 604 (which may also include a touch screen interface 606), a user located at central monitoring station 600 may change alarm settings (limits, thresholds, etc.), silence alarms, and otherwise control the individual patient monitors. User input through central station user interface 604 (or touch screen 606) are transmitted by remote control module 602 to SDC connection 207, and from there to the individual patient monitor to be controlled.

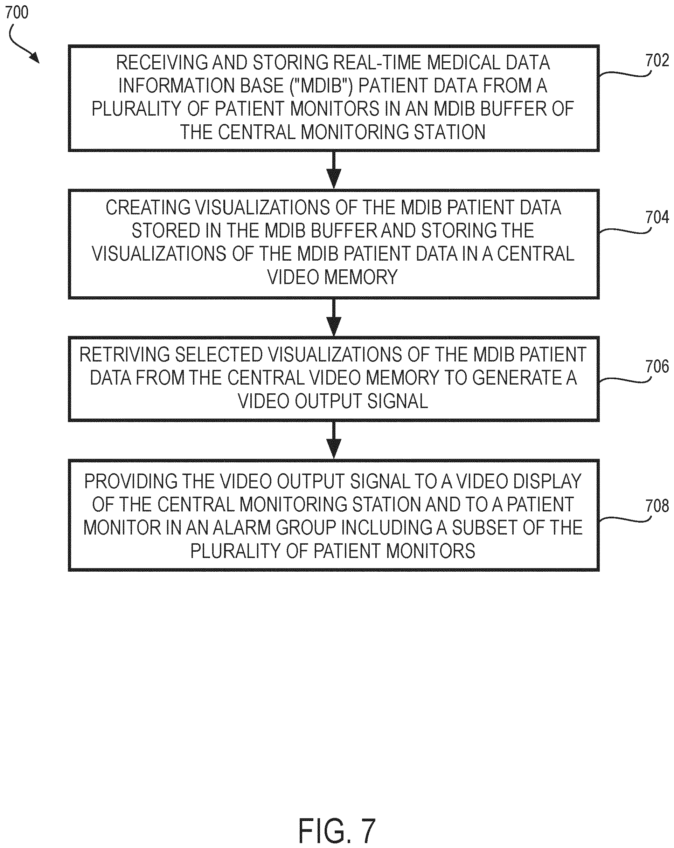

FIG. 7 is a flow diagram illustrating a method 700 for operation of a central monitoring station according to one or more embodiments. In a first step 702 of method 700, MDIB patient data from a plurality of patient monitors is received and stored in an MDIB buffer memory of the central monitoring station.

In step 704 of method 700, visualizations of the MDIB patient data stored in the MDIB buffer memory are created, and these visualizations are stored in a central video memory. In step 706, selected visualizations of the MDIB patient data stored in the central video memory are retrieved to generate a video output signal. Then, in step 708, the video output signal from step 706 is provided to a video display of the central monitoring station and to a patient monitor in an alarm group including a subset of the plurality of patient monitors.

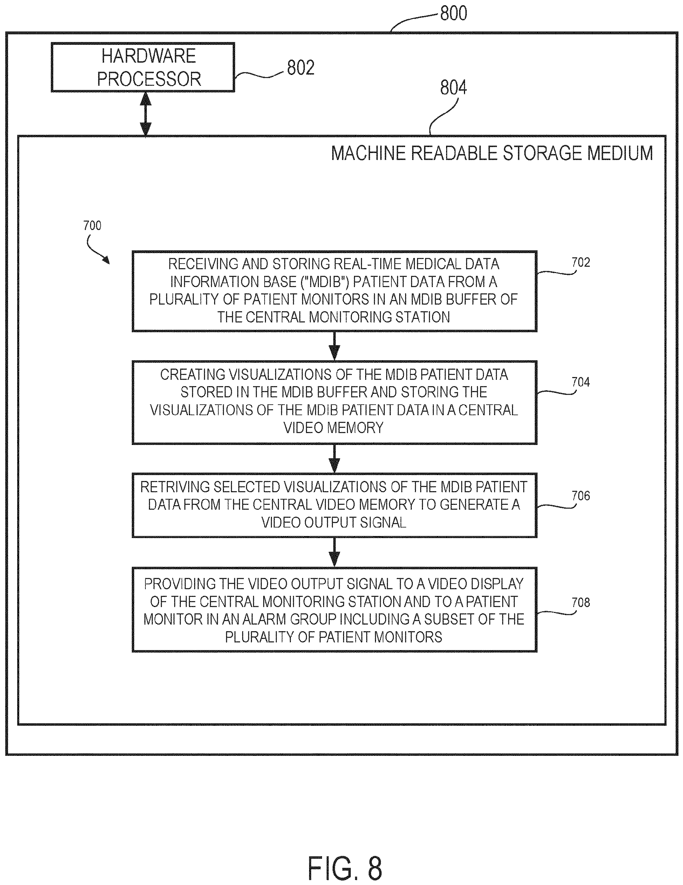

FIG. 8 is a block diagram representing a computing resource 800 implementing a method of operating a central monitoring system according to one or more examples. The computing resource 800 may be, for example and without limitation, a personal desktop computer (*e.g.,* a personal computer), a mobile computing platform (*e.g.,* a laptop or tablet), or a desktop computer accessing cloud computing resources. Computing resource 800 may include at least one hardware processor 802 and a non-transitory machine readable storage medium 804. As illustrated, machine readable storage medium 804 may store instructions, that when executed by hardware processor 802 (either directly or via emulation/virtualization), cause hardware processor 802 to perform the method 700 of operating a central monitoring station described above with reference to FIG. 7.

In various examples, hardware processor 802 may be, for example and without limitation, a microcontroller, a central processing unit ("CPU"), a digital signal processor ("DSP"), a programmed logic array ("PLA"), or a custom processing circuit. Instructions may be executed by one or more processors, such as one or more central processing units ("CPU"), digital signal processors ("DSPs)", general purpose microprocessors, application specific integrated circuits ("ASICs"), field programmable logic arrays ("FPGAs"), or other equivalent integrated or discrete logic circuitry. Accordingly, the term "processor," as used herein refers to any of the foregoing structure or any other structure suitable for implementation of the techniques described herein. In addition, in some aspects, the functionality described herein may be provided within dedicated hardware and/or software modules. Also, the techniques could be fully implemented in one or more circuits or logic elements. A "controller," including one or more processors, may use electrical signals and digital algorithms to perform its receptive, analytic, and control functions, which may further include corrective functions. Thus, a controller is a specific type of processing circuitry, comprising one or more processors and memory, that implements control functions by way of generating control signals.

A computer-readable media may be any available media that may be accessed by a computer. By way of example, such computer-readable media may comprise random access memory ("RAM"), read-only memory ("ROM"), electrically-erasable/programmable read-only memory ("EEPROM"), compact disc ROM ("CD-ROM") or other optical disk storage, magnetic disk storage or other magnetic storage devices, or any other medium that may be used to carry or store desired program code in the form of instructions or data structures and that may be accessed by a computer. Disk and disc, as used herein, includes compact disc ("CD"), laser disc, optical disc, digital versatile disc ("DVD"), floppy disk and Blu-ray^{®} disc where disks usually reproduce data magnetically, while discs reproduce data optically with lasers.

Note also that the software implemented aspects of the subject matter hereof are usually encoded on some form of program storage medium or implemented over some type of transmission medium. The program storage medium is a non-transitory medium and may be magnetic (*e.g.,* a floppy disk or a hard drive) or optical (*e.g.,* a compact disk read only memory, or "CD ROM"), and may be read only or random access. Similarly, the transmission medium may be twisted wire pairs, coaxial cable, optical fiber, or some other suitable transmission medium known to the art. The claimed subject matter is not limited by these aspects of any given implementation.

Accordingly, a first embodiment comprises a central monitoring system including a medical data information base ("MDIB") buffer memory for receiving and storing real-time MDIB patient data from a plurality of patient monitors. A software module creates visualizations of the MDIB patient data stored in the MDIB buffer memory and stores the visualizations of the MDIB patient data in a central video memory. A display image generator selectively retrieves visualization of the MDIB patient data and provides them to a display of the central monitoring system.

Further in the first embodiment, a remote video distribution device may retrieve selected visualizations of MDIB patient data from the central video memory to generate a video output signal, and provides the video output signal to a video display of the central monitoring station.

Further in the first embodiment, a remote video distribution device may be provided for retrieving selected visualizations of MDIB patient data from the central video memory to generate a video output signal, and to provide the video output signal to a video display of the central monitoring station.

Further in the first embodiment, the remote video distribution device may further provide visualization of the MDIB patient data of a first patient to a first patient monitor in an alarm group including a subset of the plurality of patient monitors.

Further in the first embodiment, the remote video distribution device may further provide visualization of the MDIB patient data of a second patient to the first patient monitor, the second patient being including the alarm group.

Further in the first embodiment, the real-time MDIB patient data may be provided to the MDIB buffer memory via a direct service-oriented device connection.

Further in the first embodiment, the real-time MDIB patient data may be provided to the MDIB buffer memory via a wireless local area network connection.

Further in the first embodiment the visualizations of MDIB patient data stored in the central video memory may include multi-modality visualizations of a plurality of patient data waveforms.

A second embodiment comprises a method of operating a central monitoring station. The central monitoring station receives and stores real-time medical data information base ("MDIB") patient data from a plurality of patient monitors in an MDIB buffer memory of the central monitoring station. The central monitoring station creates visualizations of the MDIB patient data stored in the MDIB buffer memory and stores the visualizations of the MDIB patient data in a central video memory. Selected visualizations of MDIB patient data are retrieved from the central video memory to generate a video output signal, and the video output signal is provided to a video display of the central monitoring station.

Further in the second embodiment, the method may include providing the visualizations of the MDIB patient data of a first patient to a first patient monitor in an alarm group including a subset of the plurality of patient monitors.

Further in the second embodiment, the method may include providing visualizations of the MDIB patient data of a second patient to the first patient monitor, the second patient being included in the same alarm group as the first patient.

Further in the second embodiment, the method may include providing the real-time MDIB patient data to the MDIB buffer memory via a direct service-oriented device connection.

Further in the second embodiment, the method may include providing the real-time MDIB patient data to the MDIB buffer memory via a wireless local area network connection.

Further in the second embodiment, the the visualizations of MDIB patient data stored in the central video memory may include multi-modality visualizations of a plurality of patient data waveforms.

Further in the second embodiment, the method may include generating audio alarm outputs in response to alarm status indications in the MDIB patient data stored in the MDIB buffer memory.

The term "module" is accorded herein its ordinary and accustomed meaning in the art-hardware, software, or a combination of hardware and software that implements some desired function. The interchangeability between software and hardware is well known in the art and finds applicability in technological contexts such as those disclosed herein. Thus, unless stated otherwise, a module may be implemented in, for example, electronic circuits comprised of electronic components such as transistors, resistors, inductors, capacitor, etc. Alternatively, a module may be implemented in software in the form of executable instructions stored by a processor-based resource. Alternatively, a module may be implemented in an integrated circuit such as an ASIC, an EPROM, or an EEPROM. Or, perhaps, in some combination of these choices.

The detailed description is made with reference to the accompanying drawings and is provided to assist in a comprehensive understanding of various example embodiments of the present disclosure. Changes may be made in the function and arrangement of elements discussed without departing from the spirit and scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the spirit and scope of the present disclosure.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner. Use of the phrase "exceed" in one or more embodiments, indicates that a measured value could be higher than a pre-determined threshold (*e.g.,* an upper threshold), or lower than a pre-determined threshold (*e.g.,* a lower threshold). When a pre-determined threshold range (defined by an upper threshold and a lower threshold) is used, the use of the phrase "exceed" in one or more embodiments could also indicate a measured value is outside the pre-determined threshold range (*e.g.,* higher than the upper threshold or lower than the lower threshold). The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

Various modifications to the disclosure will be readily apparent to those skilled in the art, and the generic principles defined herein may be applied to other variations without departing from the spirit or scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

This disclosure includes the subject matter set out in the following numbered Paragraphs or "Paras":
Para 1. A central monitoring station, comprising: a medical data information base ("MDIB") buffer memory for receiving and storing real-time MDIB patient data from a plurality of patient monitors; a software module for creating visualizations of the MDIB patient data stored in the MDIB buffer memory; a central video memory coupled to the software module for storing the visualizations of the MDIB patent data; and a display image generator module for selectively displaying the visualizations of MDIB patent data stored in the central video memory.
Para 2. The central monitoring station of Para 1, further comprising: a remote video distribution device for retrieving selected visualizations of MDIB patient data from the central video memory to generate a video output signal, and to provide the video output signal to a video display of the central monitoring station.
Para 3. The central monitoring station of Para 2, wherein the remote video distribution device further provides visualization of the MDIB patient data of a first patient to a first patient monitor in an alarm group including a subset of the plurality of patient monitors.
Para 4. The central monitoring station of Para 3, wherein the remote video distribution device further provides visualization of the MDIB patient data of a second patient to the first patient monitor, the second patient being included in the subset of patient monitors in the alarm group.
Para 5. The central monitoring station of Para 1, wherein the real-time MDIB patient data is provided to the MDIB buffer memory via a direct service-oriented device connection.
Para 6. The central monitoring station of Para 1, wherein the real-time MDIB patient data is provided to the MDIB buffer memory via a wireless local area network connection.
Para 7. The central monitoring station of Para 1, wherein the visualizations of MDIB patient data stored in the central video memory include multi-modality visualizations of a plurality of patient data waveforms.
Para 8. The central monitoring station of Para 1, further comprising an alarm processing module for generating audio alarm outputs in response to alarm status indications in the MDIB patient data stored in the MDIB buffer memory.
Para 9. A method of operating a central monitoring station, comprising: receiving and storing real-time medical data information base ("MDIB") patient data from a plurality of patient monitors in an MDIB buffer memory of the central monitoring station; creating visualizations of the MDIB patient data stored in the MDIB buffer memory and storing the visualizations of the MDIB patient data in a central video memory; retrieving selected visualizations of MDIB patient data from the central video memory to generate a video output signal; and providing the video output signal to a video display of the central monitoring station.
Para 10. The method of Para 9, further comprising: providing the visualizations of the MDIB patient data of a first patient to a first patient monitor in an alarm group including a subset of the plurality of patient monitors.
Para 11. The method of Para 10, further comprising: providing visualizations of the MDIB patient data of a second patient to the first patient monitor, the second patient being included in the subset of patient monitors in the alarm group.
Para 12. The method of Para 9, further comprising: providing the real-time MDIB patient data to the MDIB buffer memory via a direct service-oriented device connection.
Para 13. The method of Para 9, further comprising: providing the real-time MDIB patient data to the MDIB buffer memory via a wireless local area network connection.
Para 14. The method of Para 9, wherein the visualizations of MDIB patient data stored in the central video memory include multi-modality visualizations of a plurality of patient data waveforms.
Para 15. The method of Para 9, further comprising: generating audio alarm outputs in response to alarm status indications in the MDIB patient data stored in the MDIB buffer memory.
Para 16. A computer-readable medium tangibly embodying instructions that, when executed by a processor, performs a method of operating a central monitoring station, comprising: providing a medical data information base ("MDIB") buffer memory for receiving and storing real-time MDIB patient data from a plurality of patient monitors; creating visualizations of the MDIB patient data stored in the MDIB buffer memory and storing the visualizations of the MDIB patient data in a central video memory; retrieving selected visualizations of MDIB patient data from the central video memory to generate a video output signal; and providing the video output signal to a video display of the central monitoring station.
Para 17. The computer-readable medium of Para 16, wherein the method of operating a central monitoring station further comprises: providing visualizations of the MDIB patient data of a second patient to the first patient monitor, the second patient being included in the subset of patient monitors in the alarm group.
Para 18. The computer-readable medium of Para 16, wherein the method of operating a central monitoring station further comprises: providing the real-time MDIB patient data to the MDIB buffer memory via a direct service-oriented device connection.
Para 19. The computer-readable medium of Para 16, wherein the method of operating a central monitoring station further comprises: providing the real-time MDIB patient data to the MDIB buffer memory via a wireless local area network connection.
Para 20. The computer-readable medium of Para 16, wherein the visualizations of MDIB patient data stored in the central video memory include multi-modality visualizations of a plurality of patient data waveforms.
Para 21. The computer-readable medium of Para 16, wherein the method of operating a central monitoring station further comprises: generating audio alarm outputs in response to alarm status indications in the MDIB patient data stored in the MDIB buffer memory.

## Claims

1. A central monitoring station, comprising:
a medical data information base ("MDIB") buffer memory for receiving and storing real-time MDIB patient data from a plurality of patient monitors;
a software module for creating visualizations of the MDIB patient data stored in the MDIB buffer memory;
a central video memory coupled to the software module for storing the visualizations of the MDIB patent data; and
a display image generator module for selectively displaying the visualizations of MDIB patent data stored in the central video memory.

2. The central monitoring station of claim 1, further comprising:
a remote video distribution device for retrieving selected visualizations of MDIB patient data from the central video memory to generate a video output signal, and to provide the video output signal to a video display of the central monitoring station.

3. The central monitoring station of claim 2, wherein the remote video distribution device further provides visualization of the MDIB patient data of a first patient to a first patient monitor in an alarm group including a subset of the plurality of patient monitors.

4. The central monitoring station of claim 3, wherein the remote video distribution device further provides visualization of the MDIB patient data of a second patient to the first patient monitor, the second patient being included in the subset of patient monitors in the alarm group.

5. The central monitoring station of any one of the preceding claims, wherein the real-time MDIB patient data is provided to the MDIB buffer memory via a direct service-oriented device connection.

6. The central monitoring station of any one of the preceding claims, wherein the real-time MDIB patient data is provided to the MDIB buffer memory via a wireless local area network connection.

7. The central monitoring station of any one of the preceding claims, wherein the visualizations of MDIB patient data stored in the central video memory include multi-modality visualizations of a plurality of patient data waveforms.

8. The central monitoring station of any one of the preceding claims, further comprising an alarm processing module for generating audio alarm outputs in response to alarm status indications in the MDIB patient data stored in the MDIB buffer memory.

9. A method of operating a central monitoring station, comprising:
receiving and storing real-time medical data information base ("MDIB") patient data from a plurality of patient monitors in an MDIB buffer memory of the central monitoring station;
creating visualizations of the MDIB patient data stored in the MDIB buffer memory and storing the visualizations of the MDIB patient data in a central video memory;
retrieving selected visualizations of MDIB patient data from the central video memory to generate a video output signal; and
providing the video output signal to a video display of the central monitoring station.

10. The method of claim 9, further comprising:
providing the visualizations of the MDIB patient data of a first patient to a first patient monitor in an alarm group including a subset of the plurality of patient monitors.

11. The method of claim 10, further comprising:
providing visualizations of the MDIB patient data of a second patient to the first patient monitor, the second patient being included in the subset of patient monitors in the alarm group.

12. The method of any one of claims 9 to 11, further comprising:
providing the real-time MDIB patient data to the MDIB buffer memory via a direct service-oriented device connection and/or via a wireless local area network connection.

13. The method of any one of claims 9 to 12, wherein the visualizations of MDIB patient data stored in the central video memory include multi-modality visualizations of a plurality of patient data waveforms.

14. The method of any one of claims 9 to 13, further comprising:
generating audio alarm outputs in response to alarm status indications in the MDIB patient data stored in the MDIB buffer memory.

15. A computer-readable medium tangibly embodying instructions that, when executed by a processor, performs the method of any one of claims 9 to 14.
